# EUROPEAN PATENT SPECIFICATION

(11) **EP 1 871 906 B1**
(45) Date of publication and mention of the grant of the patent: **10.10.2012**
(21) Application number: 06740852.6
(22) Date of filing: 07.04.2006
(51) Int. Cl.: C12Q 1/68, C07H 21/02, C07H 21/04

(54) **GENOTYPING HLA LOCI**
GENOTYPISIERUNG VON HLA-LOCI
GÉNOTYPAGE DE LOCI DE HLA

(30) Priority: 08.04.2005 US 669760 P
(43) Date of publication of application: 02.01.2008
(73) Proprietor: Linkage Biosciences, Inc., San Francisco, CA 94123 (US)
(72) Inventor: ANTOVICH, Zachary, San Francisco, California 94123 (US)
(74) Representative: Sexton, Jane Helen
(86) International application number: PCT/US2006/013462
(87) International publication number: WO 2006/110735

(56) References cited:
- US-A1- 2003 165 884
- US-A1- 2004 002 073
- DOWNING J ET AL: "Five-locus HLA typing of hematopoietic stem cell donor volunteers using PCR sequence specific primers" GENETIC TESTING, LARCHMONT, NY, US, vol. 8, no. 3, 1 October 2004 (2004-10-01), pages 301-312, XP009117560 ISSN: 1090-6576
- TIEMANN C ET AL: "RAPID DNA TYPING OFHLA-B27 ALLELE BY REAL-TIME PCR USINGLIGHTCYCLER TECHNOLOGY" CLINICAL LABORATORY, CLIN LAB PUBLICATIONS, HEIDELBERG, vol. 47, no. 3-4, 1 January 2001 (2001-01-01), pages 131-134, XP008072918 ISSN: 1433-6510
- SLATEVA K ET AL: "HLA-DRB FLUOROTYPING BASED ON SYBR GREEN MEDIATED MELTING CURVES" TISSUE ANTIGENS, MUNKSGAARD, COPENHAGEN, DK, vol. 59, no. 2, SUPPL, 18 May 2002 (2002-05-18), page 13, XP001181079 ISSN: 0001-2815
- FUKUSHIMA ET AL.: 'Duplex Real Time SYBR Green PCR Assays for Detection of 17 Species of Food or Waterborne Pathogens in Stools' JOURNAL OF CLINICAL MICROBIOLOGY vol. 41, pages 5314 - 5346, XP002400106
- MONIS P T ET AL: "Comparison of SYTO9 and SYBR Green I for real-time polymerase chain reaction and investigation of the effect of dye concentration on amplification and DNA melting curve analysis", ANALYTICAL BIOCHEMISTRY, ACADEMIC PRESS INC, NEW YORK, vol. 340, no. 1, 1 May 2005 (2005-05-01), pages 24-34, XP004816986, ISSN: 0003-2697, DOI: 10.1016/J.AB.2005.01.046
- GIGLIO STEVEN ET AL: "Demonstration of preferential binding of SYBR Green I to specific DNA fragments in real-time multiplex PCR.", NUCLEIC ACIDS RESEARCH 15 NOV 2003 LNKD- PUBMED:14602929, vol. 31, no. 22, 15 November 2003 (2003-11-15), page e136, ISSN: 1362-4962
- ANONYMOUS, [Online] Retrieved from the Internet: <URL:http://www.pcrstation.com/pcr-troubles hooting/> [retrieved on 2009-11-16]
- KWOK S ET AL: "Effects of primer-template mismatches on the polymerase chain reaction: human immunodeficiency virus type 1 model studies", NUCLEIC ACIDS RESEARCH, OXFORD UNIVERSITY PRESS, SURREY, GB, vol. 18, no. 4, 25 February 1990 (1990-02-25), pages 999-1005, XP002220845, ISSN: 0305-1048
- BON MA ET AL: "GENOTYPING OF HLA-B27 BYREAL-TIME PCR WITHOUT HYBRIDIZATION PROBES", CLINICAL CHEMISTRY, AMERICAN ASSOCIATION FOR CLINICAL CHEMISTRY, WASHINGTON, DC, vol. 46, no. 7, 1 January 2000 (2000-01-01), pages 1000-1002, XP008072915, ISSN: 0009-9147
- GAFFNEY DAIRENA ET AL: "A PCR based method to determine the Kalow allele of the cholinesterase gene: The E-1-k allele frequency and its significance in the normal population", JOURNAL OF MEDICAL GENETICS, BMJ PUBLISHING GROUP, LONDON, GB, [Online] vol. 31, no. 3, 1 March 1994 (1994-03-01), pages 248-250, XP009152985, ISSN: 0022-2593 Retrieved from the Internet: URL:http://www.ncbi.nlm.nih.gov/pmc/articl es/PMC1049753/pdf/jmedgene00282 0076.pdf>
- BUNCE M ET AL: "Phototyping: comprehensive DNA typing for HLA-A, B, C, DRB1, DRB3, DRB4, DRB5 & DQB1 by PCR with 144 primer mixes utilizing sequence-specific primers (PCR-SSP)", TISSUE ANTIGENS, MUNKSGAARD, COPENHAGEN, DK, vol. 46, 1 January 1995 (1995-01-01), pages 355-367, XP008096257, ISSN: 0001-2815, DOI: 10.1111/J.1399-0039.1995.TB03127.X
- C Tse ET AL: "Quantification des acides nucléiques par PCR quantitative en temps réel", Ann Biol Clin, 1 May 2003 (2003-05-01), pages 279-293, XP55009529, Retrieved from the Internet: URL:http://www.jle.com/e-docs/00/03/FE/CF/ vers_alt/VersionPDF.pdf [retrieved on 2011-10-14]
- RIRIE K M ET AL: "PRODUCT DIFFERENTIATION BY ANALYSIS OF DNA MELTING CURVES DURING THE POLYMERASE CHAIN REACTION", ANALYTICAL BIOCHEMISTRY, ACADEMIC PRESS INC, NEW YORK, vol. 245, no. 2, 1 January 1997 (1997-01-01), pages 154-160, XP001015991, ISSN: 0003-2697, DOI: 10.1006/ABIO.1996.9916

## Description

### FIELD OF THE INVENTION

Methods for detecting nucleotide sequences and genotyping complex loci.

### BACKGROUND OF THE INVENTION

The major histocompatibility complex (MHC) of humans is a cluster of genes located on chromosome six. The Human Leukocyte Antigen (HLA) genes are located within this complex and encode cell-surface antigen-presenting proteins. These proteins play an important role in the immune system's ability to recognize "self" versus "non-sell" and are a major factor influencing immunity, autoimmunity, genetic disease, and tissue transplantation. HLA proteins are divided into three classes: Class I (HLA A, B, C, etc), Class II (HLA DR, DQ, DP, etc.), and Class III which include other components such as complement proteins and cytokines. Class I and II genes are highly polymorphic, resulting in tremendous genetic diversity across the human population.

HLA typing is a process whereby an individual's HLA protein or genotype is identified. HLA typing is commonly performed prior to solid organ transplantation. HLA matching of a patient and donor can decrease immunological rejection and improve survival outcomes (Takemoto et al, Twelve years' experience with national sharing of HLA-matched cadaveric kidneys for transplantation, The New England Journal Of Medicine, 2000 Oct 12;343(15);1078-84). HLA typing is also used in a wide variety of other applications including paternity testing, forensics analysis, genetic disease testing, and general biomedical research.

Historically, HLA typing was performed using serological techniques; however, these techniques cannot differentiate between many of the alleles known to exist in the population. Newer, DNA-based genotyping methods are more informative and are becoming widely accepted. There are three common methods of HLA genotyping: (1) Direct DNA sequencing, which requires a PCR amplification step and expensive sequencing procedures; this method is used to discover new alleles and when high resolution typing is required. (2) Sequence Specific Oligonucleotide Probe PCR (SSOP-PCR), which requires a PCR amplification step, an immobilization step, a probe detection step, and an analysis step. (3) Sequence Specific Priming PCR (SSP-PCR), which requires a PCR amplification step, a gel electrophoresis step, and an analysis step. Downing *et al.,* 2004; 8(3); 301-312 discloses five-locus HLA typing of hematopoietic stem cell donor volunteers using SSP-PCR. Tiemann *et al.,* 2002; 47; 131-134 discloses rapid DNA typing of HLA-B27 allele by real-time PCR using LightCycler technology. Slateva et al., 2002, Tissue Antigens, 59(2) supplement, 13 discloses HLA-DRB fluorotyping based on SYBR Green mediated melting curves.

All three methods are time consuming and performed by highly trained medical technologists. Direct HLA sequencing is unnecessary and too expensive for most typing needs. SSOP-PCR is labor intensive unless expensive machinery is implemented to automate procedures. SSP-PCR is also labor intensive; but may be the simplest method to perform. With SSP-PCR, the discriminating power of PCR is used to amplify specific alleles or allele groups. Oligonucleotide primers are designed to overlap polymorphic regions. Primers that fully hybridize to these regions have a higher melting temperature (Tm) than mismatched pairs and facilitate amplification. Mismatched primers fail to amplify. Genotyping an HLA locus via SSP-PCR requires multiple PCR reactions, enough to define each allele or allele group. For example, the HLA-A gene alone has 262 alleles and approximately 25 allele families as defined by Schreuder, G. et al, The HLA Dictionary 2004: a summary of HLA-A, - B, -C, -DRB 1/3/4/5 and -DQBI alleles and their association with serologically defined HLA- A, -B, -Q -DR and -DQ antigens, Tissue Antigens 2005: 65: 1-55. See also Krausa and Browning, A comprehensive PCR-SSP typing system for identification of HLA-A locus alleles, Tissue Antigens, 1996: 47: 237-144.

SSP-PCR technology is commonly used to perform HLA genotyping of cadaver organ donors. Often, this work occurs late at night when, sadly, many accident victims arrive at hospitals or morgues. Donated organs are harvested and stored on ice awaiting transplantation. Meanwhile, blood samples from cadaver donors are sent to diagnostic labs to perform HLA typing. Organ ischemia time is inversely proportional to transplant success so rapid turn-around time of HLA typing is critical. The sooner the organ can be transplanted the better. HLA typing is often the time limiting variable in this process. Post-run analysis following SSP-PCR is a lengthy process requiring gel electrophoresis, data documentation, and data evaluation. A need, therefore, exists within the art for an improved version of SSP-PCR genotyping, whereby results can be obtained more quickly, with fewer steps, in a more automated fashion.

Using the invention described herein, HLA identification occurs in a single step. DNA test samples are added to pre-filled reaction vessels and loaded onto a thermal cycler/reader. No further pipetting, handling, reading or analysis is needed. Just load the sample, run and report.

### SUMMARY OF THE INVENTION

The invention describes a novel method of genotyping within an HLA locus using a multiplex PCR system having a uniform master PCR mixture and applying a uniform thermocycling profile, the method comprising the steps of (i) establishing a plurality of at least 25 PCR reaction vessels each with an aqueous solution containing a control PCR primer pair for amplifying a control region of DNA, a different PCR HLA allele specific primer pair for amplifying different HLA alleles within the locus and a uniform master PCR mixture having a fluorescent dye able to selectively bind double stranded DNA; (ii) adding a biological sample containing HLA encoding DNA and control encoding DNA; (iii) amplifying the HLA encoding DNA and control encoding DNA in the reaction vessels using a uniform thermocycling profile across the plurality of reaction vessels to obtain solutions containing amplified DNA comprising a control amplicon and HLA amplicon and HLA amplicons, where the melting temperature of the control amplicon is at least 10°C less than the melting temperature of the HLA amplicons; and, (iv) determining the HLA type by the DNA melting profile in the solutions. The invention also describes a system for genotyping within an HLA locus using a method of the invention, where the system comprises:
i) a thermocycler comprising a plurality of at least 25 PCR reaction vessels where each reaction vessel contains a PCR primer pair able to amplify a control region of DNA, an HLA allele specific PCR primer pair for amplifying different HLA alleles within the HLA locus and a uniform master PCR mixture having a fluorescent dye able to detect double stranded DNA where the melting temperature of the control amplicon amplified by the control primer pair is at least a 10° C less than the melting temperature of the HLA amplicon amplified by the allele specific primer pair; and,
ii) a computer operably linked to the reaction vessels where the computer is able to determine the HLA genotype by comparing the melting profiles of the solutions to those of standard solutions.

### BRIEF DESCRIPTION OF THE DRAWINGS

Figure 1 - Melt curve plot (-dF/dT) of HLA-A group specific A02 alleles with Negative Control DNA sample. (Duplicate)

Figure 2 - Melt curve plot (-dF/dT) of HLA-A group specific A02 alleles with Positive DNA sample. (Duplicate)

Figure 3 - Melt curve plot (-dF/dT) of HLA-A group specific A24 alleles with Negative Control DNA sample. (Duplicate)

Figure 4 - Melt curve plot (-dF/dT) of HLA-A group specific A24 alleles with Positive DNA sample. (Duplicate)

Figure 5 - Melt curve plot (-dF/dT) ofHLA-A group specific A02 alleles with Positive DNA sample.

Figure 6 - Melt curve plot (-dF/dT) ofHLA-A group specific A02 alleles with Negative DNA sample.

Figure 7 - Melt curve plot (-dF/dT) of HLA-A group specific A7401 alleles with Positive DNA sample.

Figure 8 - Melt curve plot (-dF/dT) of HLA-A group specific A7401 alleles with Negative DNA sample. (Duplicate)

### DETAILED DESCRIPTION

### I. Introduction.

This invention provides for a simple system of PCR for genotyping an HLA locus. Prior to this work, it was unexpected that one could simultaneously amplify and then identify all the SNPs in a given HLA locus using a uniform master mix, a uniform thermocycling profile, and fluorescent melt curve analysis. The number of polymorphisms, the sequence similarity between alleles that often results in undesired co-amplification, the need for all the different primer pairs to amplify similarly, the need for each amplicon to melt at a similar temperature, the need to avoid DNA sequence that might produce extraneous melt curves, and the need to melt at temperatures that permit distinguishing between the specific allele and a control amplicon, presented a logistical roadblock to such a system. Surprisingly, it has been discovered that by using melt curve analysis, judicious selection of the mastermix and careful design of primers, one can create a system that amplifies HLA loci to a degree that permits genotyping in a single, simultaneously cycled PCR based system.

The PCR process is described in US patents 4,683,195, 4,683,202, and 4,965,188. Previous work by Newton et al demonstrated a method of genotyping using ARMS, a form of SSP-PCR (Newton et al, Analysis of any point mutation in DNA The amplification refractory mutation system (ARMS), Nucleic Acids Research 17(7): 2503-2516, 1989 and US pat. 5,595,890). This strategy has been used with real-time PCR and fluorescent melt curve analysis to detect single nucleotide polymorphisms (SNP's) (Papp, A et al, Single Nucleotide Polymorphism Genotyping Using Allele-Specific PCR and Fluorescence Melting Curves, BioTechniques Vo135, 2-6, May 2003). On the other hand, SNP detection with real-time PCR and fluorogenic probes has been more widely reported (Livak, K.J. Allelic discrimination usingfluorogenic probes and the 5'nuclease assay, Genet.Anal. 14(5-6):143-149, 1999. Several important obstacles make the former technique more difficult to design and implement than the latter.

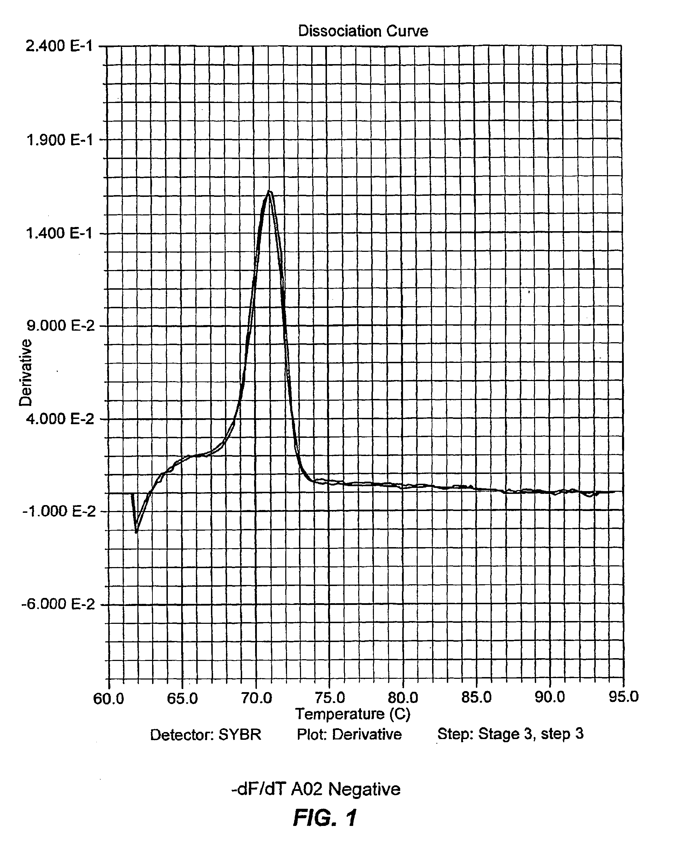

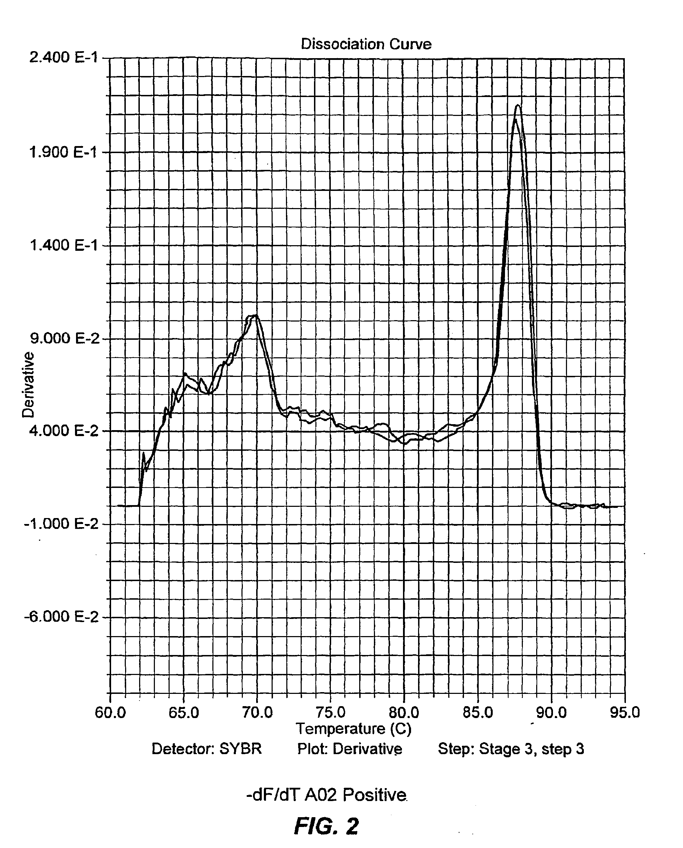

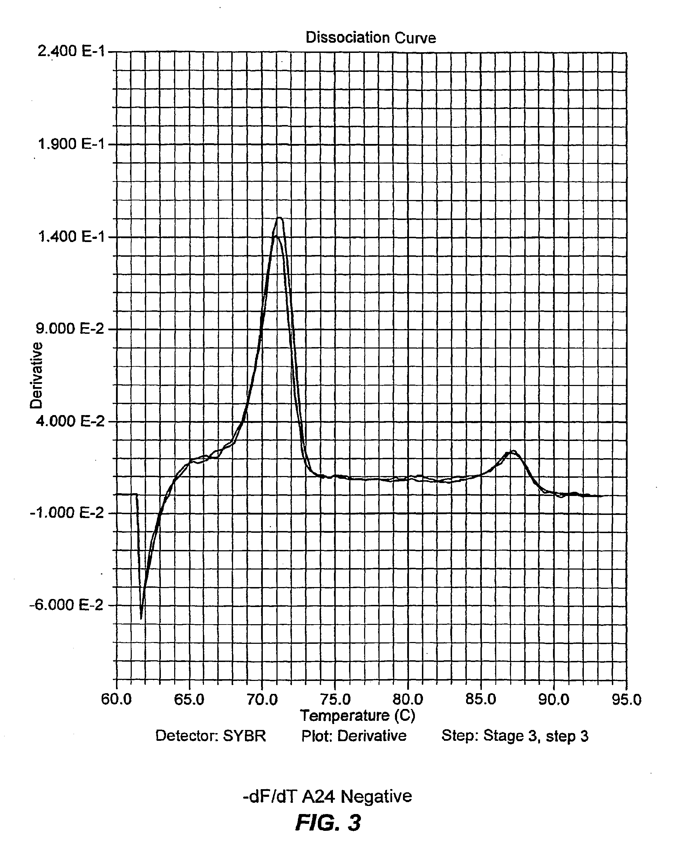

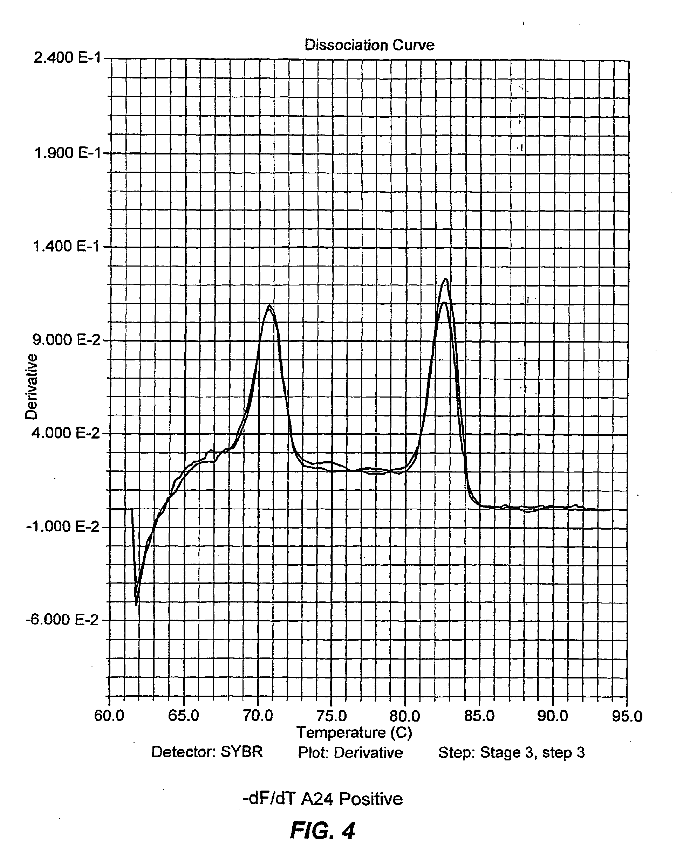

A method of melt curve analysis was published in 1993 by Tombler and Deutsch (Spectrofluorometric Assay For Hybridization of Oligodeoxynucleotides Using Ethidium Dimer, Biotechniques, Dec 1993, Volume 15, Number 6: pp 1060-1064) and later by Yguerabide and Ceballos (Quantitative Fluorescence method for continuous measurement of DNA hybridization kinetics using a fluorescent intercalator, Anal Biochem, 1995 Jul 1; 228(2) :208-220). In these papers, aqueous fluorophores selectively bind double stranded DNA (dsDNA) and fluoresce when excited at their appropriate wavelengths. This phenomenon is exploited to identify DNA by its Tm. As DNA melts, fluorescent dye is released into solution and fluorescence decreases. Differences in the Tm of individual DNA species appear as changes in the slope of the graph of fluorescence vs. temperature. For simplicity, negative first derivative analysis of fluorescence provides an easy means to identify DNA products by transforming slope changes into distinct curves (Fig. 1-4).

SNP assays using real-time PCR and fluorogenic probes are easier to design and implement because the assays require two flanking primers and a third oligonucleotide probe. Fluorescent signal is released only after all three hybridization events occur and the fluorophore on the probe is cleaved. By contrast, dsDNA binding dyes used in melt curve analysis fluoresce on contact with any dsDNA. The specificity of melt curves must be verified to confirm whether the observed fluorescence is specific or the result of unexpected amplification events. Using genomic DNA further increases the possibility that SSP primers will anneal at locations other than their intended targets. For these reasons, SSP-PCR and melt curve analysis requires more optimization and careful attention to design and remains less popular than real-time PCR methods.

Before our work, successful genotyping of a complex, highly polymorphic locus using melt curve analysis in a single thermocycling run could not be predicted. Previous published reports have demonstrated identification of a few individual SNP sites (see Papp above; also Germer and Higuchi, Single Tube Genotyping without Oligonucleotide Probes, Genome Research, 9:72-78, 1999); however, a variety of obstacles remained to successfully genotyping an entire HLA locus. These obstacles include:
(1) Unexpected melt curve effects caused by dsDNA binding dyes: The properties of some dsDNA binding dyes have been shown to bias GC bonds or melt in multiple steps with longer DNA. The properties of melt curves are significantly different from electrophoresis banding; one relies on molecular weight and the other on Tm. A PCR reaction that produces a single band on an electrophoresis gel may produce multiple bands when analyzed using melt curves and visa versa. It was unknown if these dyes would create unexpected results over a variety of SSP-PCR reactions (Giglio, et al, Demonstration of preferential binding of SYBR® Green I to specific DNA fragments in real-time multiplex PCR, Nucleic Acids Research, Vol 31, No 22, 2003).
(2) Unknown specificity issues caused by co-amplification of specific alleles and their competing targets: Allele specific PCR can often amplify competing alleles, but gel electrophoresis is generally less sensitive than spectrophotometry so a small amount of competing allele (noise) is permissible. It was unclear if this noise would pose a problem with fluorescent melt curves when targeting so many different sequences.
(3) Poor amplification and/or specificity issues caused by reagents: A single, uniform mastermix may not produce robust results across a plurality of reaction vessels. Genotyping with SSP-PCR requires numerous reactions and it would be too time consuming to optimize reagents for each individual assay. A single mastermix would be required, and it was questionable whether this would work.
(4) Poor assay performance caused by a uniform thermocycling profile: Again, it wasn't clear if a plurality of reactions could be designed around the same conditions and selectively amplify. Holding temperature consistent would limit optimization options.
(5) Interference caused by multiplexed negative control and allele specific primers: It was unclear if multiplexing these sets in a single PCR reaction would affect reproducibility or cause unacceptable primer dimer. Also, it was a challenge to design a control having a Tm sufficiently separate from every other amplicon in the genotyping set.

The outcome was not assured, but surprisingly, these obstacles were overcome, and after a significant amount of optimization and good fortune, the proof of concept was finished and a working model of HLA genotyping was developed. Design strategies are discussed below.

### II. Definitions.

Unless otherwise defined, all words have their ordinary meaning.

Alleles - Alternative forms of a genetic locus; a single allele for each locus is inherited separately from each parent.

Amplicon - A term for any small, replicating DNA fragment.

Consanguineous - Relationship by blood; descendant from a common ancestor.

First Derivative - The derivative of a function at a point measures the rate at which the function's value changes as the function's argument changes; a derivative provides a mathematical formulation of the notion of rate of change.

Fluorophore - A small molecule, or a part of a larger molecule, that can be excited by light to emit fluorescence.

Genotype - All or part of the genetic constitution of an individual or group.

Ischemia - Decreased flow of oxygenated blood to an organ due to inadequate blood flow.

Locus - The position on a chromosome of a particular gene or allele.

Mastermix - A combination of PCR reagents (e.g. MgCl, dNTP's, buffer, etc.)

Minor groove - The area of the double helix of DNA between the sugar-phosphate backbones that is less exposed as opposed to the "major groove" which is more exposed.

Polymerase chain reaction - A method for amplifying a DNA sequence using a heat- stable polymerase and two oligonucleotide primers, one complementary to the sense strand at one end of the sequence to be amplified and the other complementary to the antisense strand at the other end. Because the newly synthesized DNA strands can subsequently serve as additional templates for the same primer sequences, successive rounds of primer annealing, strand elongation, and dissociation produce rapid amplification of the desired sequence.

Polymorphisms - The occurrence in a population (or among populations) of several phenotypic forms associated with alleles of one gene or homologs of one chromosome.

Thermal melting temperature (Tm) - The temperature below which an oligonucleotide primer will anneal to its DNA template and above which the oligonucleotide primer will dissociate from its DNA template.

Thermocycler - An instrument that repeatedly cycles through various temperatures required for an iterative, temperature-dependant chemical process such as PCR.

Uniform - having always the same form, manner, or degree: not varying or variable.

### Abbreviations

PCR - Polymerase chain reaction

SNP - Single nucleotide polymorphism

HLA - Human Leukocyte Antigen

. SSP-PCR - sequence-specific-primer polymerase chain reaction

SSOP-PCR - sequence-specific-oligonucleotide probes polymerase chain reaction

dsDNA - Double stranded DNA

ssDNA - Single stranded DNA

### III. Sample preparation.

Genomic DNA samples are obtained from human blood. A variety of published protocols or commercial kits are available to isolate DNA from whole blood or its separated components. A generic, laboratory method using heparinized vacutainers and phenol extraction, is equally effective (The Nucleic Acid Protocols Handbook, March 2000, Pages 3-7). The fluorescence absorbance should be measured at 260nm and 280nm to determine DNA purity.

Genomic DNA can be stored at 4°C if the sample is to be used within a few days. It should be frozen at -20°C if the sample will be stored for more than a week. For extended storage time, use a -70°C freezer.

### IV. Systems for HLA typing.

The individual PCR reactions are performed simultaneously in individual tubes or in thermocycling reaction plates. 96-well and 384-well reaction plates have both been used in the current disclosure.

Reaction vessels contain the chemical components, polymerase enzyme, buffer, and [0049] Reaction vessels contain the chemical components, polymerase enzyme, buffer, and oligonucleotide primers required for DNA amplification. They are placed into a PCR thermocycler, which heats and cools the reaction vessels in order to amplify DNA using the PCR process. A variety of commercial manufacturers sell PCR thermocyclers. In the present disclosure, the 7900HT sequence detection system from Applied Biosystems is used.

The individual PCR reactions are analyzed at the termination of the amplification process. A fluorescent, double-stranded, DNA binding dye is used to identify the presence or absence of amplification products. A thermocycling instrument coupled to a computer that monitors and stores fluorescence data is used to detect these products. Again, a variety of commercial manufacturers sell computer controlled PCR thermocyclers that are capable of monitoring and recording fluorescence (e.g. Applied Biosystems, Bio-Rad, and Stratagene). Note that the reaction vessels can be run on a standard PCR thermocycler and then moved to a second thermocycler that monitors fluorescence at the end of run or both amplification and post-amplification detection can be performed on the same thermocycler. Fluorescence recording is not required during PCR thermocycling. The test is an end-point read, requiring a melt curve recording after the amplification process. In the present disclosure , the 7900HT sequence detection system from Applied Biosystems coupled to a Dell computer is used to both amplify and detect specific products.

Fluorescence is monitored and saved onto a computer during the detection process. Afterward, software is used to calculate the first derivative of the fluorescence data. The specific amplification products are identified from the graph of the negative first derivative (-dF) vs. temperature (dT). The first derivative calculations can be performed onboard the computer used for detection or the raw data can be downloaded and the first derivative calculations can be done on a separate computer >Ririe KM, Rasmussen RP, Wittwer CT. Product differentiation by analysis of DNA melting curves during the polymerase chain reaction. Anal Biochem 1997; 245:154-60).

### V. Creating a large scale multiplex-multivessel PCR system using a uniform mastermix and thermocycling profile.

The followings is disclosed :
PCR reagents and dsDNA binding dye:

Standard PCR reagents are optimized and combined to form a mixture that will be used in all reaction vessels. These reagents include PCR buffer, MgCl, dNTP's, and DNA polymerase. Individual PCR components can be titrated to determine the most effective combination. Multiple thermocycling runs are required to determine an efficient balance of reagents. Selecting an appropriate DNA polymerase is important. A *Taq* DNA polymerase (*Thermus aquaticus*) or its equivalent with moderate processivity, low error rate, and lacking 3' to 5' exonuclease activity is sufficient. The reagents are optimized with a subset of PCR primers that are designed following the guidelines below. Once the components are tested and optimized, they are added together to form a "mastermix". The mastermix is used in all reaction vessels and minimizes liquid handling procedures.

A double stranded DNA binding dye is tested and added to the mastermix. In the present disclosure, SYBR^{®} Green I from Molecular Probes is used, although, LC Green^{®} from Idaho Technologies, ethidium bromide, or other similar, dsDNA-binding dye can be substituted. SYBR^{®} Green I is excited at 497nm and emits at 520nm (Molecular Probes Product Information Sheet, SYBR® Green I Nucleic Acid Gel Stain, Revised Sept 24, 2003).

The disclosure utilizes several design strategies to avoid uncertainties associated with DNA binding dyes: (1) Whenever possible, amplicons are designed to be short since longer amplicons might produce additional melt curves. A "short" amplicon is around 100 base pairs. The allele specific product should not overlap the negative control product (2) The negative control amplicon is designed to have a low Tm so that it melts early and allows greater flexibility when designing allele specific products (discussed below). (3) All primers are tested to ensure strong signal and reproducible results. Primers that fail are redesigned and retested in a feedback loop process. (4) The optimized mastermix and thermocycling conditions are held constant during the primer design process.

### Thermocycling conditions:

PCR thermocycling temperatures, ramp rates, hold times, and cycle numbers should be empirically tested with a subset of allelic specific primers to maximize signal strength and minimize time requirements. Numerous tests may be required to determine the most effective profile. For example, three step PCR profiles (anneal, extend, melt) can be tested versus two step profiles (anneal and extend at same temp, then melt). Changes can be made to annealing, extension and melting temperatures as well as to hold times and cycle duration. The total cycle number for each run should be altered during the optimization process to associate the best thermocycling profile with the optimum cycle number. Fewer cycles mean quicker genotyping. Melt curve analysis can be used to determine which profiles and cycle numbers produce the most robust melt curves (highest amplitude) and have the best reproducibility. After the optimum thermocycling profile and cycle number have been determined they should remain constant during the remaining development process.

### Negative Control:

A negative control PCR primer set is used to eliminate the possibility that a failed PCR reaction could be mistaken for a negative amplification (false negative). The control amplicon in many traditional PCR assays is often larger than the product of interest. In these applications, agarose gel electrophoresis is used to separate heterogeneous DNA by size. Smaller DNA species move more quickly through the gel than larger species; thus, it may be advantageous to design a larger control amplicon since specific DNA products will appear more quickly on a gel, and electrophoresis can be terminated soon after the control band appears.

In the present disclosure, however, the control amplicon is designed to be smaller than the allele specific amplicon. The negative control is also designed to be AT rich, which will produce a melt curve at a much lower temperature than the allele specific products with which it is multiplexed. Thus, the negative control curve, will be detected at an earlier temperature and not interfere with allele-specific products. DNA length and composition are two important variables in Tm. Smaller DNAs are more affected by changes in length than larger DNA's. The larger the product, the closer it approaches its Tm limit. Using an AT rich, small control amplicon decreases the probability of overlap with allele specific amplicons and allows greater design flexibility; allele specific products can be designed to melt at any temperature above the negative control.

Apo B was selected in the present disclosure to be used as the negative control. This single copy gene is highly studied, and extensive sequence information is available (Fisher and Ginsberg, Complexity in the Secretory Pathway: The Assembly and Secretion of Apolipoprotein B-containing Lipoproteins, The Journal of Biological Chemistry, Vol.277, No.20, May 17, pp17377-17380, 2002; see also GenBank accession number M14162). Several small, AT-rich regions devoid of polymorphisms and repeating units were selected and primers were designed using primer design software (Steve Rozen and Helen J. Skaletsky, Primer3 on the WWW for general users and for biologist programmers. In: Krawetz S, Misener S (eds) Bioinformatics Methods and Protocols: Methods in Molecular Biology. Humana Press, Totowa, NJ, 2000, pp 365-386).

Primer design depends on many factors. For a review please see Abd-Elsalam, K. African Journal of Biotechnology Vol. 2 (5), pp. 91-95, May 2003 or Kolmodin and Williams, The Nucleic Acid Protocols Handbook, PCR: Basic Principles and Routine Practice, March 2000, Pages 572-580. For a review of thermodynamic calculations see Breslauer et al., (1986) Proc. Nat. Acad. Sci. 83:3746-50 and Sugimoto et al., (1996) Nucl. Acids Res. 24:4501-4505. Oligonucleotide primers can be ordered from a commercial supplier (e.g. IDT, Sigma Genosys, Invitrogen, etc.) or synthesized using the appropriate equipment and chemistries.

Once a working negative control primer set is developed, it should be tested against normal DNA samples. Forward and reverse primer concentrations should be varied between 0nM and 900nM, creating an optimization matrix for each primer set. Non-template control (lacking DNA) reactions are set up to detect primer dimer amplification at various primer concentrations. The primer sets with the most robust signal and minimal or undetectable primer dimer amplification should be selected.

### HLA Allele Specific Primer Design Strategies:

The following primer design strategies are critical to creating a successful multiplexed, multi-vessel, multi-PCR system for HLA genotyping. There are four key steps: (1) picking primer binding sites; (2) designing primers and incorporating 3' mismatched bases when appropriate; (3) testing the primers to determine selectivity and reproducibility; and (4) redesigning primers that fail.

(1) Picking primer binding sites: Hundreds of alleles have been identified at the HLA-A locus alone. Primer sites must be carefully chosen that will include the allele or group of alleles targeted but exclude homologous or closely related sequence. An allele group or single allele shall be defined as an "allele" for simplicity in the following explanations. The disclosure makes use of SSP PCR so polymorphisms on the 3' end of primer sites are targeted and used to amplify single alleles versus their closely related, nearly identical homologs.

Recent advances in bioinformatics have created a number of useful tools that compare sequence homology and identify polymorphisms. GenBank, BLAST, dbSNP, and other resources are available from the National Center for Biotechnology Information (NCBI). Additionally, the Anthony Nolan trust offers a number of HLA-specific bioinformatics tools. All published alleles within the HLA-A loci are obtained from the public domain and aligned via sequence alignment tools such as ClustalX (Thompson,J.D., Gibson,T.J., Plewniak,F., Jeanmougin,F. and Higgins,D.G. The ClustalX windows interface: flexible strategies for multiple sequence alignment aided by quality analysis tools. Nucleic Acids Research, 24:4876-4882, 1997) or even by hand. Areas of similarity between alleles and areas of polymorphism should be visible upon alignment. Primer binding sites are chosen so that each primer pair includes the allele of interest but excludes all other alleles.

Known homologous genes such as HLA-A, B, C, E, F, G, etc. for class I loci and pseudo genes HLA H, J, K, etc. should be compared against the selected primer binding sites to ensure specificity. The same rule applies to class II loci. If the selected polymorphic sites are shared at another locus, new sites must be chosen. Non-specific amplification can be eliminated with this additional bioinformatics step.

(2) Primer design: Primers are designed with any number of tools publicly and commercially available following traditional rules of PCR. For difficult primer sets that produce non-specific amplification, two strategies can improve assay success rates. The first is to incorporate an internal mismatch on the 3' end of primers to increase allelic discrimination. Mismatches are incorporated as needed following primer testing. Kwok, S. et al describe mismatch effects and PCR efficiency (Effects of primer-template mismatches on the polymerase chain reaction: Human immunodeficiency virus type 1 model studies, Nucleic Acids Research, Vol. 18, No.4 1990,999-1005. Primer sets are designed with a Tm of approximately 58°C-62°C. Mismatches can be incorporated at the penultimate 3' base, two bases from the end, and occasionally three bases from the end. The mismatches can be randomly picked and empirically tested, although certain rules should be followed when possible. For example, picking a mismatch identical to the competitive allele is often successful (e.g. pick a "C" if the allele to be excluded is a "C" at the same position).

The second strategy is to add a 5' extended tail to the primer(s). An additional 5-7 GC bases can have a marked effect on amplification. A product that was previously slow to amplify can be accelerated using this technique. The tail will produce a longer hybridizing area in later PCR rounds, which increases the Tm and promotes annealing and extension.

The primer sets should be compared to the full sequence of the human genome using BLAST or a similar program to ensure non-specific hybridization sites do not exist elsewhere (Altschul, S.F., Gish, W., Miller, W., Myers, E.W. & Lipman, D.J. (1990) Basic local alignment search tool. J. Mol. Biol. 215:403-410). Also, Alu sequence, transposons, and other repeating elements should be tested in silico. Designs are rejected if non-specific sites are discovered.

(3) Primer testing and optimization: Finally, primers are tested against positive and negative control DNA. Positive control DNA must be obtained or identified in advance. A concentration matrix should be set up for each primer pair (25nM to 900nM) to determine the optimal concentrations. Non-template control reactions are added to identify non-specific amplification. A second primer concentration matrix is set up to optimize the multiplex reaction consisting of group specific primers and negative control primers. Non-template controls are run to identify non-specific cross priming events.

Primers must satisfy the following empirical criteria:
1. Positive DNA control sample: An allele-specific melt curve must be observed at the appropriate Tm window. Spurious curves should not overlap the Tm window. The negative control peak may also be visible, but this is not a requirement.
2. Negative DNA control sample: A melt curve is observed for the negative control amplicon only. A curve is not observed within the allele-specific Tm window.
3. Positive or negative DNA control sample: spurious curves caused by false priming or primer dimer, if visible, do not interfere with positive and negative results.

Assays fail for the following reasons:
1. False Negative: A melt curve is observed for the negative control amplicon only. The assay fails because the allele of interest is not detected. Specific PCR may have failed. DNA tertiary structure, Tm issues, intentional 3'mismatches, and bad reagents can all cause assay failure.
2. False Positive: Melt curves are observed for the negative control amplicon and the positive control amplicon. In this case, both primer sets were detected but only the negative product should have amplified. Failed assays of this type are not uncommon in development since primer binding sites may differ by as little as a single base pair. In these cases, redesigning the primer sets and incorporating an intentional 3' mismatch may provide additional specificity.
3. No call: Spurious amplification products (false priming) and/or primer dimer products detected and overlapping the Tm of specific products.

Failed primers must be redesigned. Simple changes to primer length or base pair composition may be sufficient. Alternatively, new primer binding sites must be chosen and the primers re-designed following the steps above.

Primer pairs that pass initial screening are tested extensively with multiple positive and negative control samples to determine assay accuracy and reproducibility.

### Multiplex-multivessel PCR system:

Once all the allele specific primers have been designed and optimized, they are mixed with negative control primers and transferred to the appropriate reaction vessels. DNA sample, polymerase and mastermix are mixed together and added to each vessel. The vessels are ready for thermocycling.

### VI. Genotyping an HLA locus

In the current disclosure, 30 reactions are desired to type the HLA-A locus. The vessels (plates or tubes) are transferred to the thermocycling instrument which is programmed with the optimized thermocycling profile and cycle number. At the end of the amplification process, the vessels are cooled briefly then slowly heated to begin the melt cycle. Fluorescence measurements are recorded and stored by the computer. Raw fluorescence and temperature data is downloaded from the thermocycling instrument and the negative first derivative is calculated (-dF). -dF is plotted against temperature to generate a curve for each reaction vessel which is printed and stored.

Positive DNA is identified by Tm-specific peaks (the negative peak may or may not be present). Negative control DNA is identified by a single peak at the correct Tm window. The pattern of peaks over the sum of the reaction vessels defines the HLA locus type. All reaction vessels should show, at a minium, one amplification peak: either the negative or allele specific peak. If neither peak is present, the PCR reaction has failed in that vessel.

Once the PCR process has been verified in each well, the vessels showing group specific amplification products are recorded. Typically, each DNA has two group specific products (one maternal, one paternal) in non-homozygous, non-consanguineous DNA. Of course, occasionally, DNA samples may be homozygous or share group specific alleles, in which case a single, group specific curve is detected out of the full set. This curve may have a greater amplitude than normal since twice as much DNA is present prior to amplification.

Because of the complexity of the HLA genes, exceptions often exist within group specific primers sets. For example, certain group-specific alleles may not amplify with the rest of the group. These rare cases should be detected with separate primer sets if the application requires fine resolution. In another example, certain non-group-specific alleles may amplify as group alleles. These rare types should be noted and may produce ambiguous genotyping results. Additional reactions can elucidate ambiguities; however the added time and expense testing for rare alleles may not be practical. Public internet sites such as ncbi.nlm.nih.gov/projects/mhc can be queried to determine allele frequencies.

HLA nomenclature (an example of a common HLA-A allele):

A*02 - first two digits indicate family or serology group name.

A*0202 - distinct allele

A*020202 - synonymous base change (non-coding).

A*02020201 - intronic base change.

(Schreuder, G. et al, The HLA Dictionary 2004: a summary of HLA-A, -B, -C, - DRB1/3/4/5 and -DQB1 alleles and their association with serologically defined HLA-A, -B, - C, -DR and -DQ antigens, Tissue Antigens 2005: 65: 1-55.

As can be appreciated from the disclosure provided above, the present invention has a wide variety of applications. Accordingly, the following example is offered for illustration purposes and is not intended to be construed as a limitation on the invention in any way.

### EXAMPLE

### Example 1: HLA-A locus typing method

### Example 1A: Sample preparation:

Human genomic DNA was prepared from whole blood using heparinized vacutainers and phenol extraction (The Nucleic Acid Protocols Handbook; March 2000, Pages 3-7). HLA-A typing was performed using commercially available kits from Pel-Freeze. Additional HLA-A defined DNA samples were obtained from the European Collection of Cell Cultures (Wiltshire, U.K.). DNA samples were stored at -20°C until ready for use.

### Example 1B: Mechanical systems used to perform the invention.

The 7900HT sequence detection system from Applied Biosystems and Dell desktop computer was used for thermocycling and fluorescent detection (SDS software version 2.1). 384-well, optically clear, thermocycling reaction plates from Applied Biosystems were used as reaction vessels and covered with optically clear adhesive covers prior to thermocycling.

### Example 1C: Materials and Methods

2X Mastermix: Buffer (6.0mM MgCl2, 20mM Tris-Cl pH 8.3, 100mM KCl, 0.02% Tween 20, 1.2% glycerol, dNTP's 0.4mM dATP, 0.4mM dCTP, 0.4mM dGTP, and 0.8mM dUTP), AmpliTaq^{®} DNA polymerase (Applied Biosystems) 0.125U/uL, and SYBR^{®} Green I diluted to 1/40,000 (Molecular Probes Cat#S-7563)

10uL of 2X mastermix is transferred to each well. Primers, DNA sample, and ddH₂O (if necessary) are added to bring the final reaction volume to 20uL. The sample concentration is 1ng/uL or 20ng total DNA.

A two step PCR thermocycling profile is used to optimize primer pairs and when testing sample DNA: Heat to 95C for 90 seconds; then 38 cycles at 95°C 15sec and 64°C for 60sec (anneal and extension combined). After the run, the temperature is lowered to 4°C for 60 seconds then the melt curve cycle begins. The temperature rises from 60°C to 95°C, ramping at a rate of 2°C per minute.

As discussed, ApoB was chosen as the negative control; it is a well characterized single copy gene. Multiple, short, AT-rich regions were selected and primer sets were designed and synthesized (Integrated DNA Technologies, Inc; Coralville, IA). A single set that produced a consistent, clean melt curve at a low Tm was selected.

30 group specific primer sets were designed following the procedures outlined previously. Primer sites covering the majority of the HLA-A locus were identified from published sources. Alleles were grouped by common polymorphisms contained within exons 2 and 3 of the HLA-A gene. Shorter amplicons were preferred whenever possible to increase PCR efficiency and improve melt curve analysis. Primers were designed using Primer Express Software (Applied Biosystems), targeting a Tm between 58°- 62°C. After verifying the primer sites were specific and not shared by other homologous sequence or pseudogenes and BLASTing the set against the human genome, primers were ordered from IDT. Some primers incorporate a 3' mismatch to increase specificity. Others have an additional 5' tail to increase efficiency. Through careful planning and design, approximately 80% of our primers work the first time. The rest must be redesigned. Primer concentrations were optimized for each new set by varying both forward and reverse primer concentrations from [50nM] to [900nM]. Negative control primers were held constant at [75nM]. Optimization helped reduce false priming and often improved melt curve amplitude. Primer concentration should be optimized for each lot of new primer.
At the end of each run, raw fluorescence data was downloaded and negative first derivative values calculated to generate melt curves.

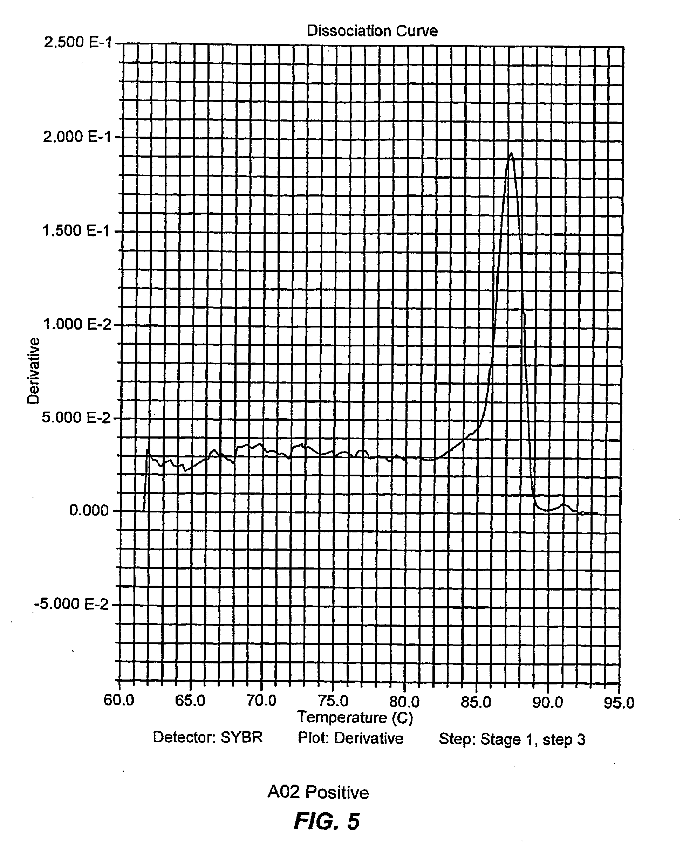

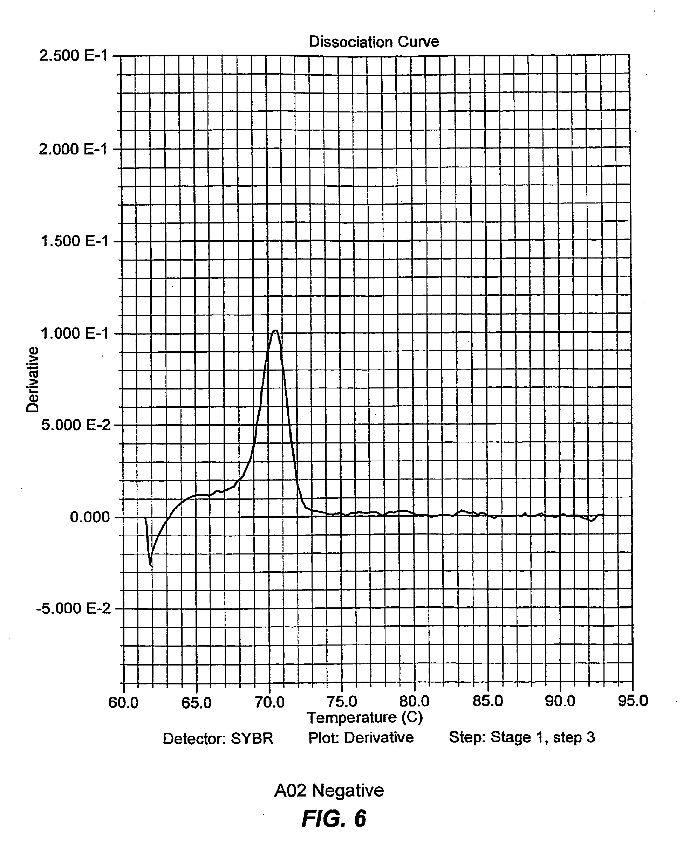

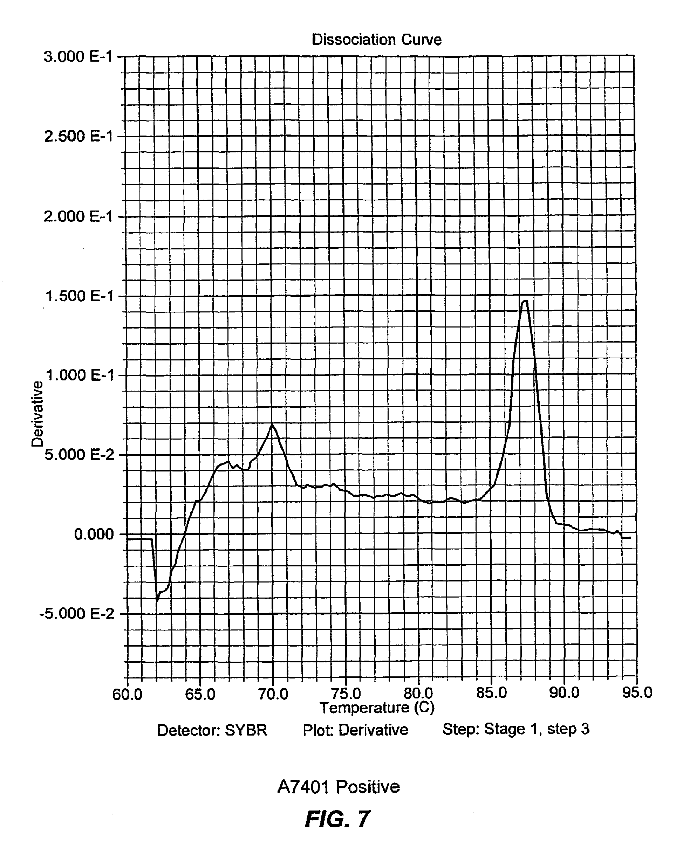

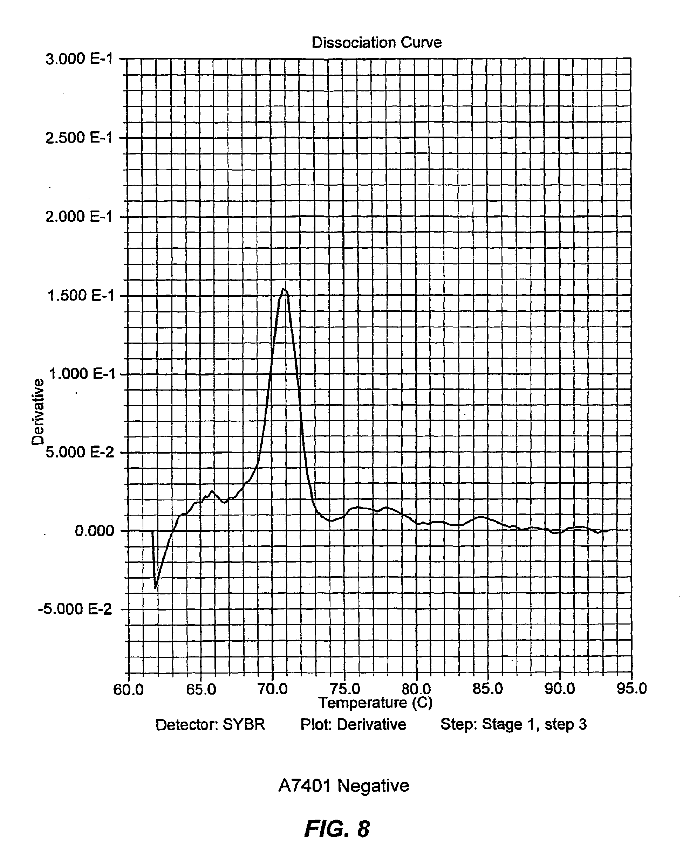

Examples of representative melt curves are provided (Fig. 1-8)

### Example 1D: Results

Primer Details:
1. A01
   F: GAGCTTATGCGCACGGTACGCAAGTGGGAGGCGAT (SEQ ID NO:1)
   R: CAGGTATCTGCGGAGCACG (SEQ ID NO:2)
   Sequences these primers will detect:
   A*010101∼09
   Sequences NOT detected:
   A*0110
   Other alleles detected:
   None
   Primer concentration: [200nM]
   Control: [50nM]
   Amplicon Length: 102bp's
   Estimated Tm = 86C
2. A02
   F: GGGAGCCCCGCTTCATCGTA (SEQ ID NO:3)
   R: TCTCCCCGTCCCAATACTCCGAA (SEQ ID NO:4)
   Sequences these primers will detect:
   A*02010101~0273 (see exceptions) Sequences NOT detected:
   A*020109,0248,0250,0255,0242?
   Other alleles detected: None
   Primer concentration: [200nM]
   Control: [75nM]
   Amplicon Length: 133bp's
   Estimated Tm = 87.26C
34. A02Part II
   F:GAGGGCCGTGCGACGTGGGGTCGGAAT (SEQ ID NO:5)
   R:GCACTTGTGCTTGGTGGTCTGAGAT (SEQ ID NO:6)
   Sequences these primers will detect: A*02010101~0272
   Sequences NOT detected:
   A*0210, 0219, 0239, 0250, 0252, 0265, and 0273 Other alleles detected:
   A*6901
   Look at melt curves for 2414, and 36's, 11's, or 68's to see power of discrimination with one primer only.
   Primer concentration: [200nM]
   Control: [75nM]
   Amplicon Length: 147bp's
   Estimated Tm = 86C
4. A0250
   F:GCTCCCACTCCATGAGGTATTTGTT (SEQ ID NO:7)
   F2:AGagGGCTCTCACTCCATGAGGTATTTGTT (SEQ ID NO:8)
   R:GGGCCGCCTCCCACTTCT (SEQ ID NO:9)
   R2:ATGGGCCGTCTCCCACTTCT (SEQ ID NO:10)
   Sequences these primers will detect:
   A*0250 and other 02.
   Sequences NOT detected:
   A*0205, 020601, 020602, 020603, 0208, 0210, 0214, 0219, 0221, 0228, 0241, 0244, 0251, 0254, 0257, 0261, 0265, 0272
   Primer concentration: [400nM] (mixture of both forward)
   Control: [75nM] (mixture of both reverse)
   Amplicon Length: 450bp's
   Estimated Tm = 90C
5. A03
   F:CCCAGTCACAGACTGACCGAGTTG (SEQ ID NO:11)
   R:CACTCCACGCACGTGTCA (SEQ ID NO:12)
   Sequences these primers will detect:
   A*03 alleles 03010101, 03010102N, 03010103, 030102, 030103, 0302, 0303N, 0304, 0306,
   0307, 0308?, 0309?, 0311N, 0312, 0313, 0314
   Sequences NOT detected:
   A*0305, 0310 (check 0308?, 0309?)
   Other alleles detected:
   A*6819
   Primer concentration: [200nM]
   Control: [75nM]
   Amplicon Length: 536bp's
   Estimated Tm = 90C
6. A03II
   F:GCTCCCACTCCATGAGGTATTTGTT (SEQ ID NO:13)
   R:GTCCACTCGGTCAGTCTGTGTC (SEQ ID NO:14)
   Sequences these primers will detect:
   A*03 alleles 03010101, 03010102N, 03010103, 030102, 030103, 0302, 0303N, 0304, 0305,
   0306, 0307, 0309, 0310, 0311N, 0313, 0314
   Sequences NOT detected:
   A*0308, 0309 questionable, and 0312
   Other alleles detected:
   A*0234, *023501, *0256, and *0262, HLA-C01
   Primer concentration: [200nM]
   Control: [100nM]
   Amplicon Length: 231bp's
   Estimated Tm = 89.8C
7. A11
   F:CCAGGTTCTCACACCATCCAGCTA (SEQ ID NO:15)
   R:GGCCCTCCAGGTAGGCTCTGT (SEQ ID NO:16)
   Sequences these primers will detect:
   A*110101 ∼ 07/09 ∼ 19
   Sequences NOT detected:
   A* 1108
   Other alleles detected:
   A*0302, *0310
   Primer concentration: [200nM]
   Control: [75nM]
   Amplicon Length: 220bp's
   Estimated Tm = 88.9C
8. A11II
   F:CCAGGTTCTCACACCATCCAGCTA (SEQ ID NO:17)
   R:ACTTGCGCTTGGTGATCTGAGGT (SEQ ID NO:18)
   Sequences these primers will detect:
   A*110101 ~ 19
   Sequences NOT detected:
   None
   Other alleles detected:
   A*01 (except 0103), A*3601, A*3602, A*3604
   Primer concentration: [200nM]
   Control: [75nM]
   Amplicon Length: 172bp's
   Estimated Tm = 88.0C
9. A23
   F:CGGAGTATTGGGACGAGGAGACTG (SEQ ID NO:19)
   R:GCCCGGCCCTCTCAACTGCTCCGCCACTC (SEQ ID NO:20)
   Sequences these primers will detect:
   A*2301~2307N, 2308N, 2310, 2311N, 2312
   Sequences NOT detected:
   A*2309
   Other alleles detected:
   A*3007, 3108, 3205
   Primer concentration: [200nM]
   Control: [75nM]
   Amplicon Length: 543bp's
   Estimated Tm = 90.0C
10. A23II
   F:CGGAGTATTGGGACGAGGAGACTG (SEQ ID NO:21)
   R:GTGGCAGGGCCTTGCCGTCGTAGGCGAA (SEQ ID NO:22)
   Sequences these primers will detect:
   A*2301~12
   Sequences NOT detected:
   A*2309
   Other alleles detected:
   A*0246, 0248, 0270, 24 (except, 240204?, 2408, 2417, 2424, 2429, 2439?, 2441, 2442)
   Primer concentration: [400nM]
   Control: [75nM]
   Amplicon Length: 445bp's
   Estimated Tm = 90.1C
11. A24
   F:TTCTCACACCCTCCAGATGATGCT (SEQ ID NO:23)
   R:CGCCTCCCACTTGCGCCT (SEQ ID NO:24)
   Sequences these primers will detect:
   A*24020101∼04/06∼13/17∼23/25∼46
   Sequences NOT detected
   A*2405, 2414, 2415, 2424
   Other alleles detected:
   A*0210, 021701, 021702, 0239
   Primer concentration: [200nM]
   Control: [75nM]
   Amplicon Length: 175bp's
   Estimated Tm = 87.2C
12. A24II
   R:GGGACGAGGAGACAGGGAGA (SEQ ID NO:25)
   L:TTGTAGTAGCGGAGCGCGA (SEQ ID NO:26)
   Sequences these primers will detect:
   A*24020101∼24020102L/240202∼06/2405∼08/2409N/10/11N/13∼15/17/18/20∼23/25∼27/29
   ∼35/36N/37∼39/40N/41∼43/45N/46; 2408? 2429? 2442?
   Sequences NOT detected:
   A*2404, 2419, 2424, 2428, 2444
   Other Alleles detected:
   A*2301∼06/2307N/2308N/2310/2311N/2312 (missing A*2309 only)
   A*3108?, 3205? AND 3107?
   Primer concentration: [200nM]
   Control: [75nM]
   Amplicon Length: 79bp's
   Estimated Tm = 82.2C
13. A24III
   F:CGGAGTATTGGGACGAGGAGACTG (SEQ ID NO:27)
   R:CTCTCTGCTGCTCCGCCACCT (SEQ ID NO:28)
   Sequences these primers will detect:
   A*A11 24 except those listed below:
   Sequences NOT detected
   A*2406?, 2408, 2413?, 2418?, 2422, 2424, 2429, 2442
   Other alleles detected:
   A*0246?, 0248?, 0270?
   Primer concentration: [200nM]
   Control: [75nM]
   Amplicon Length: 544bp's
   Estimated Tm = 90.0C
14. A2424
   F:CGGAGTATTGGGACCGGAAC (SEQ ID NO:29)
   R:CTCTCTGCTGCTCCGCCACCT (SEQ ID NO:30)
   Sequences these primers will detect:
   A*2424
   Sequences NOT detected
   None
   Other alleles detected:
   A*6826
   Primer concentration: [200nM]
   Control: [75nM]
   Amplicon Length: 588bp's
   Estimated Tm = 90.3C
15. A25
   F:CCCACTCACAGACTGACCGAGATAG (SEQ ID NO:31)
   R:CGCGCACCCGATGTAATCCTTGCCGTCGTCA (SEQ ID NO:32)
   Sequences these primers will detect:
   A*250101, 250102?, 2502, 2503, 2504
   Sequences NOT detected:
   None
   Other alleles detected:
   None
   Primer concentration: [400nM]
   Control: [75nM]
   Amplicon Length: 409bp's
   Estimated Tm = 90.2C
16. A26
   F:CCACTCACAGACTGACCGAGCTAA (SEQ ID NO:33)
   F2:CCAGTCACAGACTGACCGAGCTAA (SEQ ID NO:34)
   R:CGCGCACCCGATGTAATCCTTGCCGTCGTCA (SEQ ID NO:35)
   Sequences these primers will detect:
   A*2601, 2602, 2604, 2605?, 260701, 260702, 2608, 2609, 2610, 2611N, 2612, 2613, 2614,
   2615, 2616, 2617, 2618, 2619, 2620, 2622, and 2623
   Sequences NOT detected:
   A*2603, 2606, 2621
   Other alleles detected:
   A*4301
   Primer concentration: [400nM]
   Control: [75nM]
   Amplicon Length: 408bp's
   Estimated Tm = 90.2C
17. A26II
   F:CGGAGTATTGGGACCGGAAC (SEQ ID NO:36)
   R:GATGTAATCCTTGCCGTCGTCA (SEQ ID NO:37)
   Sequences these primers will detect:
   A*2601, 2602, 2603, 2604, 2605, 2606, 2608, 2609, 2610, 2611N, 2612, 2613, 2614, 2615,
   2616, 2617, 2618, 2620, 2621, 2622, and 2623
   Sequences NOT detected:
   A*260701, 260702, 2619
   Other alleles detected:
   A*2501~04, *3401~06, *6601~04
   Primer concentration: [200nM]
   Control: [75nM]
   Amplicon Length: 445bp's
   Estimated Tm = 90.2C
18. A29
   F:GGCTCCCACTCCATGAGGTATTTGA (SEQ ID NO:38)
   R:TCGCGGCGTCGCTGTTA (SEQ ID NO:39)
   Sequences these primers will detect:
   A*29010101 ∼ A*2911 (ALL)
   Sequences NOT detected
   None
   Other Alleles detected
   None
   Primer concentration: [200nM]
   Control: [75nM]
   Amplicon Length: 124bp's
   Estimated Tm = 87.8C
19. A30
   F:CCAGGTTCTCACACCATCCAGCTA (SEQ ID NO:40)
   R:GCCGTCGTAGGCGTGCTCT (SEQ ID NO:41)
   Sequences these primers will detect:
   A*300101~A*3012
   Sequences NOT detected:
   None
   Other Alleles detected:
   None
   Primer concentration: [200nM]
   Control: [75nM]
   Amplicon Length: 91bp's
   Estimated Tm = 84.0C
20. A31
   F:GGCTCCCACTCCATGAGGTATTTGA (SEQ ID NO:42)
   R:CGCGGAGTGGTCTCCTGGTCCCAATACTCAGGCAT (SEQ ID NO:43)
   Sequences these primers will detect:
   A*310102/3102∼07/09/10
   Sequences NOT detected
   A*3108
   Other Alleles detected
   None
   Primer concentration: [200nM]
   Control: [75nM]
   Amplicon Length: 186bp's
   Estimated Tm = 89.5C
21. A3108
   F:GGCTCCCACTCCATGAGGTATTTGA (SEQ ID NO:44)
   R:TTGTAGTAGCGGAGCGCGA (SEQ ID NO:45)
   Sequences these primers will detect:
   A*3108
   Sequences NOT detected:
   None
   Other Alleles detected:
   A*3107, B0802?, B0803?
   Primer concentration: [200nM]
   Control: [75nM]
   Amplicon Length: 186bp's
   Estimated Tm = 89.5C
22. A32
   F:CACGCAGTTCGTGCGGTCT (SEQ ID NO:46)
   R:TTGTAGTAGCGGAGCGCGA (SEQ ID NO:47)
   Sequences these primers will detect:
   A*3201∼03/05∼08
   Sequences NOT detected:
   A*3204
   Other Alleles detected:
   None
   Primer concentration: [400nM]
   Control: [75nM]
   Amplicon Length: 168bp's
   Estimated Tm = 87.5C
23. A3204 F:GCTCCCACTCCATGAGGTATTTGTT (SEQ ID NO:48)
   R:TTGTAGTAGCGGAGCGCGA (SEQ ID NO:49)
   Sequences these primers will detect:
   A*3201~06/08
   Sequences NOT detected:
   A*3207
   Other Alleles detected:
   A*2503
   Primer concentration: [400nM]
   Control: [75nM]
   Amplicon Length: 256bp's
   Estimated Tm = 89.8C
24. A34
   F:GCGGGAGCAGGCGGGTACCAGCAGGACGAT (SEQ ID NO:50)
   F2:GCGGGAGCAGTCGGGTACCGGCAGGACGAT (SEQ ID NO:51)
   R:GAGCCACTCCACGCACGT (SEQ ID NO:52)
   Sequences these primers will detect:
   A*3401~3406 (all)
   Sequences NOT detected:
   None
   Other Alleles detected:
   A*2609, 3103, 3104
   Primer concentration: F+F2=[800nM] R=[200nM]
   Control: [150nM]
   Amplicon Length: 172bp's
   Estimated Tm = 87.8C
25. A4301
   F:GGCCGGAGTATTGGGACCTAC (SEQ ID NO:53)
   R:CGATGTAATCCTTGCCGTCGTCA (SEQ ID NO:54)
   Sequences these primers will detect:
   A*4301
   Sequences NOT detected:
   None
   Other Alleles detected:
   None
   Primer concentration: [200nM]
   Control: [75nM]
   Amplicon Length: 448bp's
   Estimated Tm = 90.3C
26. A66
   F:CCCAGTCACAGACTGACCGAGTTG (SEQ ID NO:55)
   R:AGCCACTCCACGCACCG (SEQ ID NO:56)
   Sequences these primers will detect: A*6601, 6604
   Sequences NOT detected:
   A*6602, A*6603
   Other Alleles detected:
   A*0238, 11 (except 1104 and 1117), 2603, 2606, 2621
   B*1543?, 2725?, 5303?
   Primer concentration: [200nM]
   Control: [75nM]
   Amplicon Length: 540bp's
   Estimated Tm = 90.0C
27. A6602/03
   F:CGGGTACCAGCAGGACGAT (SEQ ID NO:57)
   R:GGAGCCACTCCACGCACTC (SEQ ID NO:58)
   Sequences these primers will detect:
   A*6602, 6603
   Sequences NOT detected:
   A*6601, 6604
   Other Alleles detected:
   None
   Primer concentration: [200nM]
   Control: [75nM]
   Amplicon Length: 173bp's
   Estimated Tm = 88.0C
28. A6901
   F:CGGAGTATTGGGACCGGAA (SEQ ID NO:59)
   R:CGCGGAGGAAGCGCGA (SEQ ID NO:60)
   Sequences these primers will detect:
   A*6901
   Sequences NOT detected:
   None
   Other Alleles detected:
   A*0255
   Primer concentration: [200nM]
   Control: [75nM]
   Amplicon Length: 406bp's
   Estimated Tm = 91.0C
29. A74
   F:CACGCAGTTCGTGCGGTCT (SEQ ID NO:61)
   R:CGCAGGGTCCCCAGGTCTA (SEQ ID NO:62)
Sequences these primers will detect:
   A*7401∼7409
   Sequences NOT detected:
   A*7410
   Other Alleles detected:
   None
   Primer concentration: [200nM]
   Control: [75nM]
   Amplicon Length: 156bp's
   Estimated Tm = 87.0C
30. A8001
   F:GCTCCCACTCCATGAGGTATTTGTT (SEQ ID NO:63)
   R:GCGCCCGGCGGCGTCGCTGTCGAAAT (SEQ ID-NO:64)
   Sequences these primers will detect:
   A*8001
   Sequences NOT detected:
   None
   Other Alleles detected:
   None
   Primer concentration: [400nM]
   Control: [75nM]
   Amplicon Length: 128bp's
   Estimated Tm = 88.0C

## Claims

1. A method of genotyping within an HLA locus using a multiplex PCR system having a uniform master PCR mixture and applying a uniform thermocycling profile, the method comprising the steps of:
i) establishing a plurality of at least 25 PCR reaction vessels each with an aqueous solution containing a PCR primer pair for amplifying a control region of DNA, a different PCR HLA allele specific primer pair for amplifying different HLA alleles within the locus and a uniform master PCR mixture having a fluorescent dye able to detect double stranded DNA;
ii) adding a biological sample containing HLA encoding DNA and control encoding DNA;
iii) amplifying the HLA encoding DNA and control encoding DNA in the reaction vessels using a uniform thermocycling profile across the plurality of reaction vessels to obtain solutions containing amplified DNA comprising a control amplicon and HLA amplicons, where the melting temperature of the control amplicon is at least 10° C less than the melting temperature of the HLA amplicons; and,
iv) determining the HLA type by the DNA melting profile in the solutions.

2. A method of claim 1 where the fluorescent label binds to the minor groove of the amplified double stranded DNA.

3. A method of claim 1 where the control amplicon has a Tm of below 75°C.

4. A method of claim 1 where the number of reaction vessels is sufficient to identify enough polymorphisms to define an allele family or a single allele for a given HLA locus.

5. A method of claim 1 wherein the determining step involves calculating the first derivative of the fluorescent melting profile of the solutions.

6. A method of claim 1 where the method comprises the step of using a computer to calculate the first derivative from the raw data and providing graphical results.

7. A method of claim 1 where the biological sample is from cadaveric tissue.

8. A method of claim 1 where the number of PCR reaction vessels is greater than 35.

9. A method of claim 1 where the locus is selected from the group consisting of HLA A, HLA B, HLA C, HLA DR, HLA DP, and HLA DQ.

10. A system for genotyping within an HLA locus using a method of claim 1, where the system comprises:
i) a thermocycler comprising a plurality of at least 25 PCR reaction vessels where each reaction vessel contains a PCR primer pair able to amplify a control region of DNA, an HLA allele specific PCR primer pair for amplifying different HLA alleles within the HLA locus and a uniform master PCR mixture having a fluorescent dye able to detect double stranded DNA where the melting temperature of the control amplicon amplified by the control primer pair is at least a 10° C less than the melting temperature of the HLA amplicon amplified by the allele specific primer pair; and,
ii) a computer operably linked to the reaction vessels where the computer is able to determine the HLA genotype by comparing the melting profiles of the solutions to those of standard solutions.

11. A system of claim 10 where the fluorescent dye binds to the minor groove of the amplified double stranded DNA.

12. A system of claim 10 wherein the computer determines HLA genotype by calculating the first derivative of the fluorescent melting profile of the solutions.

13. A system of claim 10 where the control amplicon has a Tm of below 75°C.

14. A system of claim 10 where the number of reaction vessels is sufficient to identify enough polymorphisms to define an allele family or a single allele for a given HLA locus.

15. A system of claim 10 where the computer calculates the first derivative of the melting profiles and provides graphical results.

16. A system of claim 10 where the number of PCR reaction vessels is greater than 35.

17. A system of claim 10 where the locus is selected from the group consisting of HLA A, HLA B, HLA C, HLA DR, HLA DP, and HLA DQ.

18. A system of claim 10 where the computer provides a uniform thermocycling profile across the plurality of reaction vessels.

## Patentansprüche

1. Verfahren zur Genotypisierung innerhalb eines HLA-Locus unter Verwendung eines Multiplex-PCR-Systems, das eine einheitliche Master-PCR-Mischung hat und Anwenden eines einheitlichen Thermocycling-Profils, wobei das Verfahren die Schritte umfasst:
1) Herstellen einer Vielzahl von mindestens 25 PCR-Reaktionsgefäßen, jedes mit einer wässrigen Lösung, die ein PCR-Primer-Paar zur Amplifizierung einer DNA-Kontrollregion, ein anderes PCR-HLA-Allel-spezifisches Primer-Paar zur Amplifizierung anderer HLA-Allele innerhalb des Locus und eine einheitliche Master-PCR-Mischung, die einen fluoreszierenden Farbstoff besitzt, der dazu in der Lage ist, doppelsträngige DNA nachzuweisen, enthält;
2) Hinzufügen einer biologischen Probe, die HLAkodierende DNA und Kontrolle-kodierende DNA enthält;
3) Amplifizieren der HLA-kodierenden DNA und Kontrolle kodierenden DNA in den Reaktionsgefäßen unter Verwendung eines einheitlichen Thermocycling-Profils über die Vielzahl von Reaktionsgefäßen, um Lösungen zu erhalten, die amplifizierte DNA enthalten, umfassend ein Kontroll-Amplicon und HLA-Amplicons, wobei die Schmelztemperatur des Kontroll-Amplicons mindestens 10°C weniger als die Schmelztemperatur der HLA-Amplicons ist; und,
4) Bestimmen des HLA-Typs durch das DNA-Schmelzprofil in den Lösungen.

2. Verfahren gemäß Anspruch 1, wobei die fluoreszierende Markierung an die kleine Furche der amplifizierten doppelsträngigen DNA bindet.

3. Verfahren gemäß Anspruch 1, wobei das Kontroll-Amplicon eine Tm von weniger als 75°C hat.

4. Verfahren gemäß Anspruch 1, wobei die Anzahl von Reaktionsgefäßen ausreicht, um genügend Polymorphismen zu identifizieren, um eine Allel-Familie oder ein einziges Allel für einen bestimmten HLA-Locus zu definieren.

5. Verfahren gemäß Anspruch 1, wobei der Bestimmungsschritt Berechnen des ersten abgeleiteten Wertes des fluoreszierenden Schmelzprofils der Lösungen beinhaltet.

6. Verfahren gemäß Anspruch 1, wobei das Verfahren den Schritt des Verwendens eines Computers umfasst, um den ersten abgeleiteten Wert von den Rohdaten zu berechnen und grafische Ergebnisse bereitstellt.

7. Verfahren gemäß Anspruch 1, wobei die biologische Probe aus Kadaver-Gewebe ist.

8. Verfahren gemäß Anspruch 1, wobei die Anzahl an PCR-Reaktionsgefäßen größer als 35 ist.

9. Verfahren gemäß Anspruch 1, wobei der Locus ausgewählt ist aus der Gruppe, bestehend aus HLA-A, HLA-B, HLA-C, HLA-DR, HLA-DP und HLA-DQ.

10. System zur Genotypisierung innerhalb eines HLA-Locus unter Verwendung eines Verfahrens gemäß Anspruch 1, wobei das System umfasst:
1) Einen Thermocycler, umfassend eine Vielzahl von mindestens 25 PCR-Reaktionsgefäßen, wobei jedes Reaktionsgefäß ein PCR-Primer-Paar, das dazu in der Lage ist, eine DNA-Kontrollregion zu amplifizieren, ein HLA-Allel-spezifisches PCR-Primer-Paar zur Amplifizierung verschiedener HLA-Allele innerhalb des HLA-Locus und eine einheitliche Master-PCR-Mischung, die einen fluoreszierenden Farbstoff besitzt, der dazu in der Lage ist, doppelsträngige DNA nachzuweisen, enthält, wobei die Schmelztemperatur des Kontroll-Amplicons, das von dem Kontroll-Primer-Paar amplifiziert wird, mindestens 10°C weniger ist als die Schmelztemperatur des HLA-Amplicons, das von dem Allel-spezifischen Primer-Paar amplifiziert wird; und
2) einen Computer, funktional verknüpft mit den Reaktionsgefäßen, wobei der Computer dazu in der Lage ist, den HLA-Genotyp durch Vergleichen der Schmelzprofile der Lösungen mit denen von Standard-Lösungen zu bestimmen.

11. System gemäß Anspruch 10, wobei der fluoreszierende Farbstoff die kleine Furche der amplifizierten doppelsträngigen DNA bindet.

12. System gemäß Anspruch 10, wobei der Computer HLA-Genotyp durch Berechnen des ersten abgeleiteten Wertes des fluoreszierenden Schmelzprofils der Lösungen bestimmt.

13. System gemäß Anspruch 10, wobei das Kontroll-Amplicon eine Tm von weniger als 75°C hat.

14. System gemäß Anspruch 10, wobei die Anzahl an Reaktionsgefäßen ausreicht, um genügend Polymorphismen zu identifizieren, um eine Allel-Familie oder ein einziges Allel für einen gegebenen HLA-Locus zu definieren.

15. System gemäß Anspruch 10, wobei der Computer den ersten abgeleiteten Wert der Schmelzprofile berechnet und grafische Ergebnisse bereitstellt.

16. System gemäß Anspruch 10, wobei die Anzahl an PCR-Reaktionsgefäßen größer als 35 ist.

17. System gemäß Anspruch 10, wobei der Locus ausgewählt ist aus der Gruppe, bestehend aus HLA-A, HLA-B, HLA-C, HLA-DR, HLA-DP und HLA-DQ.

18. System gemäß Anspruch 10, wobei der Computer ein einheitliches Thermocycling-Profil über die Vielzahl von Reaktionsgefäßen bereitstellt.

## Revendications

1. Méthode de génotypage au sein d'un locus de HLA en utilisant un système de PCR multiplex ayant un mélange de PCR maître uniforme et en appliquant un profil de thermocyclage uniforme, la méthode comprenant les étapes consistant à :
i) établir une pluralité d'au moins 25 réacteurs de PCR chacun avec une solution aqueuse contenant une paire d'amorces de PCR pour l'amplification d'une région d'ADN de contrôle, une paire d'amorces de PCR spécifiques d'allèles HLA différents pour l'amplification d'allèles HLA différents au sein du locus et un mélange de PCR maître uniforme ayant un colorant à fluorescence capable de détecter un ADN double brin ;
ii) ajouter un échantillon biologique contenant un ADN codant pour HLA et un ADN codant pour la région de contrôle ;
iii) amplifier l'ADN codant pour HLA et l'ADN codant pour la région de contrôle dans les réacteurs en utilisant un profil de thermocyclage uniforme sur la pluralité de réacteurs pour obtenir des solutions contenant l'ADN amplifié comprenant un amplicon de contrôle et des amplicons HLA, où la température de fusion de l'amplicon de contrôle est d'au moins 10 °C inférieure à la température de fusion des amplicons HLA ; et
iv) déterminer le type HLA par le profil de fusion d'ADN dans les solutions.

2. Méthode selon la revendication 1, dans laquelle le marqueur à fluorescence se lie au sillon mineur de l'ADN double brin amplifié.

3. Méthode selon la revendication 1, dans laquelle l'amplicon de contrôle a une Tm inférieure à 75 °C.

4. Méthode selon la revendication 1, dans laquelle le nombre de réacteurs est suffisant pour identifier suffisamment de polymorphismes afin de définir une famille d'allèles ou un allèle unique pour un locus de HLA donné.

5. Méthode selon la revendication 1, dans laquelle l'étape de détermination implique le calcul du premier dérivé du profil de fusion à fluorescence des solutions.

6. Méthode selon la revendication 1, dans laquelle la méthode comprend l'étape consistant à utiliser un ordinateur pour calculer le premier dérivé à partir des données brutes et à fournir des résultats graphiques.

7. Méthode selon la revendication 1, dans laquelle l'échantillon biologique provient d'un tissu cadavérique.

8. Méthode selon la revendication 1, dans laquelle le nombre de réacteurs PCR est supérieur à 35.

9. Méthode selon la revendication 1, dans laquelle le locus est choisi dans le groupe consistant en HLA A, HLA B, HLA C, HLA DR, HLA DP et HLA DQ.

10. Système de génotypage au sein d'un locus de HLA en utilisant la méthode selon la revendication 1, dans lequel le système comprend :
i) un thermocycleur comprenant une pluralité d'au moins 25 réacteurs PCR où chaque réacteur contient une paire d'amorces de PCR capables d'amplifier une région d'ADN de contrôle, une paire d'amorces de PCR spécifiques d'allèles HLA pour l'amplification d'allèles HLA différents au sein du locus de HLA et un mélange de PCR maître uniforme ayant un colorant à fluorescence capable de détecter un ADN double brin où la température de fusion de l'amplicon de contrôle amplifié par la paire d'amorces de contrôle est d'au moins 10 °C inférieure à la température de fusion de l'amplicon HLA amplifié par la paire d'amorces spécifiques d'allèles ; et,
ii) un ordinateur lié opérationnellement aux réacteurs dans lequel l'ordinateur est capable de déterminer le génotype HLA par comparaison des profils de fusion des solutions à ceux des solutions standard.

11. Système selon la revendication 10, dans lequel le colorant à fluorescence se lie au sillon mineur de l'ADN double brin amplifié.

12. Système selon la revendication 10, dans lequel l'ordinateur détermine le génotype HLA par calcul du premier dérivé du profil de fusion à fluorescence des solutions.

13. Système selon la revendication 10, dans lequel l'amplicon de contrôle a une Tm inférieure à 75 °C.

14. Système selon la revendication 10, dans lequel le nombre de réacteurs est suffisant pour identifier suffisamment de polymorphismes afin de définir une famille d'allèles ou un allèle unique pour un locus de HLA donné.

15. Système selon la revendication 10, dans lequel l'ordinateur calcule le premier dérivé des profils de fusion et fournit des résultats graphiques.

16. Système selon la revendication 10, dans lequel le nombre de réacteurs PCR est supérieur à 35.

17. Système selon la revendication 10, dans lequel le locus est choisi dans le groupe consistant en HLA A, HLA B, HLA C, HLA DR, HLA DP et HLA DQ.

18. Système selon la revendication 10, dans lequel l'ordinateur fournit un profil de thermocyclage uniforme sur la pluralité de réacteurs.


### SEQUENCE LISTING

<110> Antovich, Zachary
   Linkage Biosciences Inc.
<120> Genotyping HLA Loci
<130> 025757-000110PC
<140> PCT/US06/Not yet assigned
   <141> Not yet assigned
<150> US 60/669,760
   <151> 2005-04-08
<160> 64
<170> FastSEQ for Windows Version 4.0
<210> 1
   <211> 35
   <212> DNA
   <213> Artificial Sequence
<220>
   <223> HLA-A group specific PCR primer A01 F
<400> 1
   gagcttatgc gcacggtacg caagtgggag gcgat 35
<210> 2
   <211> 19
   <212> DNA
   <213> Artificial Sequence
<220>
   <223> HLA-A group specific PCR primer A01 R
<400> 2
   caggtatctg cggagcacg 19
<210> 3
   <211> 20
   <212> DNA
   <213> Artificial Sequence
<220>
   <223> HLA-A group specific PCR primer A02 F
<400> 3
   gggagccccg cttcatcgta 20
<210> 4
   <211> 23
   <212> DNA
   <213> Artificial Sequence
<220>
   <223> HLA-A group specific PCR primer A02 R
<400> 4
   tctccccgtc ccaatactcc gaa 23
<210> 5
   <211> 27
   <212> DNA
   <213> Artificial Sequence
<220>
   <223> HLA-A group specific PCR primer A02Part II F
<400> 5
   gagggccgtg cgacgtgggg tcggaat 27
<210> 6
   <211> 25
   <212> DNA
   <213> Artificial Sequence
<220>
   <223> HLA-A group specific PCR primer A02Part II R
<400> 6
   gcacttgtgc ttggtggtct gagat 25
<210> 7
   <211> 25
   <212> DNA
   <213> Artificial Sequence
<220>
   <223> HLA-A group specific PCR primer A0250 F
<400> 7
   gctcccactc catgaggtat ttgtt 25
<210> 8
   <211> 30
   <212> DNA
   <213> Artificial Sequence
<220>
   <223> HLA-A group specific PCR primer A0250 F2
<400> 8
   agagggctct cactccatga ggtatttgtt 30
<210> 9
   <211> 18
   <212> DNA
   <213> Artificial Sequence
<220>
   <223> HLA-A group specific PCR primer A0250 R
<400> 9
   gggccgcctc ccacttct 18
<210> 10
   <211> 20
   <212> DNA
   <213> Artificial Sequence
<220>
   <223> HLA-A group specific PCR primer A0250 R2
<400> 10
   atgggccgtc tcccacttct 20
<210> 11
   <211> 24
   <212> DNA
   <213> Artificial Sequence
<220>
   <223> HLA-A group specific PCR primer A03 F
<400> 11
   cccagtcaca gactgaccga gttg 24
<210> 12
   <211> 18
   <212> DNA
   <213> Artificial Sequence
<220>
   <223> HLA-A group specific PCR primer A03 R
<400> 12
   cactccacgc acgtgtca 18
<210> 13
   <211> 25
   <212> DNA
   <213> Artificial Sequence
<220>
   <223> HLA-A group specific PCR primer A03II F
<400> 13
   gctcccactc catgaggtat ttgtt 25
<210> 14
   <211> 22
   <212> DNA
   <213> Artificial Sequence
<220>
   <223> HLA-A group specific PCR primer A03II R
<400> 14
   gtccactcgg tcagtctgtg tc 22
<210> 15
   <211> 24
   <212> DNA
   <213> Artificial Sequence
<220>
   <223> HLA-A group specific PCR primer A11 F
<400> 15
   ccaggttctc acaccatcca gcta 24
<210> 16
   <211> 21
   <212> DNA
   <213> Artificial Sequence
<220>
   <223> HLA-A group specific PCR primer A11 R
<400> 16
   ggccctccag gtaggctctg t 21
<210> 17
   <211> 24
   <212> DNA
   <213> Artificial Sequence
<220>
   <223> HLA-A group specific PCR primer A11II F
<400> 17
   ccaggttctc acaccatcca gcta 24
<210> 18
   <211> 23
   <212> DNA
   <213> Artificial Sequence
<220>
   <223> HLA-A group specific PCR primer A11II R
<400> 18
   acttgcgctt ggtgatctga ggt 23
<210> 19
   <211> 24
   <212> DNA
   <213> Artificial Sequence
<220>
   <223> HLA-A group specific PCR primer A23 F
<400> 19
   cggagtattg ggacgaggag actg 24
<210> 20
   <211> 29
   <212> DNA
   <213> Artificial Sequence
<220>
   <223> HLA-A group specific PCR primer A23 R
<400> 20
   gcccggccct ctcaactgct ccgccactc 29
   <210> 21
   <211> 24
   <212> DNA
   <213> Artificial Sequence
<220>
   <223> HLA-A group specific PCR primer A23II F
<400> 21
   cggagtattg ggacgaggag actg 24
<210> 22
   <211> 28
   <212> DNA
   <213> Artificial Sequence
<220>
   <223> HLA-A group specific PCR primer A23II R
<400> 22
   gtggcagggc cttgccgtcg taggcgaa 28
<210> 23
   <211> 24
   <212> DNA
   <213> Artificial Sequence
<220>
   <223> HLA-A group specific PCR primer A24 F
<400> 23
   ttctcacacc ctccagatga tgct 24
<210> 24
   <211> 18
   <212> DNA
   <213> Artificial Sequence
<220>
   <223> HLA-A group specific PCR primer A24 R
<400> 24
   cgcctcccac ttgcgcct 18
<210> 25
   <211> 20
   <212> DNA
   <213> Artificial Sequence
<220>
   <223> HLA-A group specific PCR primer A24II R
<400> 25
   gggacgagga gacagggaga 20
<210> 26
   <211> 19
   <212> DNA
   <213> Artificial Sequence
<220>
   <223> HLA-A group specific PCR primer A24II L
<400> 26
   ttgtagtagc ggagcgcga 19
<210> 27
   <211> 24
   <212> DNA
   <213> Artificial Sequence
<220>
   <223> HLA-A group specific PCR primer A24III F
<400> 27
   cggagtattg ggacgaggag actg 24
<210> 28
   <211> 21
   <212> DNA
   <213> Artificial Sequence
<220>
   <223> HLA-A group specific PCR primer A24III R
<400> 28
   ctctctgctg ctccgccacc t 21
<210> 29
   <211> 20
   <212> DNA
   <213> Artificial Sequence
<220>
   <223> HLA-A group specific PCR primer A2424 F
<400> 29
   cggagtattg ggaccggaac 20
<210> 30
   <211> 21
   <212> DNA
   <213> Artificial Sequence
<220>
   <223> HLA-A group specific PCR primer A2424 R
<400> 30
   ctctctgctg ctccgccacc t 21
<210> 31
   <211> 25
   <212> DNA
   <213> Artificial Sequence
<220>
   <223> HLA-A group specific PCR primer A25 F
<400> 31
   cccactcaca gactgaccga gatag 25
<210> 32
   <211> 31
   <212> DNA
   <213> Artificial Sequence
<220>
   <223> HLA-A group specific PCR primer A25 R
<400> 32
   cgcgcacccg atgtaatcct tgccgtcgtc a 31
<210> 33
   <211> 24
   <212> DNA
   <213> Artificial Sequence
<220>
   <223> HLA-A group specific PCR primer A26 F
<400> 33
   ccactcacag actgaccgag ctaa 24
<210> 34
   <211> 24
   <212> DNA
   <213> Artificial Sequence
<220>
   <223> HLA-A group specific PCR primer A26 F2
<400> 34
   ccagtcacag actgaccgag ctaa 24
<210> 35
   <211> 31
   <212> DNA
   <213> Artificial Sequence
<220>
   <223> HLA-A group specific PCR primer A26 R
<400> 35
   cgcgcacccg atgtaatcct tgccgtcgtc a 31
<210> 36
   <211> 20
   <212> DNA
   <213> Artificial Sequence
<220>
   <223> HLA-A group specific PCR primer A26II F
<400> 36
   cggagtattg ggaccggaac 20
<210> 37
   <211> 22
   <212> DNA
   <213> Artificial Sequence
<220>
   <223> HLA-A group specific PCR primer A26II R
<400> 37
   gatgtaatcc ttgccgtcgt ca 22
<210> 38
   <211> 25
   <212> DNA
   <213> Artificial Sequence
<220>
   <223> HLA-A group specific PCR primer A29 F
<400> 38
   ggctcccact ccatgaggta tttga 25
<210> 39
   <211> 17
   <212> DNA
   <213> Artificial Sequence
<220>
   <223> HLA-A group specific PCR primer A29 R
<400> 39
   tcgcggcgtc gctgtta 17
<210> 40
   <211> 24
   <212> DNA
   <213> Artificial Sequence
<220>
   <223> HLA-A group specific PCR primer A30 F
<400> 40
   ccaggttctc acaccatcca gcta 24
<210> 41
   <211> 19
   <212> DNA
   <213> Artificial Sequence
<220>
   <223> HLA-A group specific PCR primer A30 R
<400> 41
   gccgtcgtag gcgtgctct 19
<210> 42
   <211> 25
   <212> DNA
   <213> Artificial Sequence
<220>
   <223> HLA-A group specific PCR primer A31 F
<400> 42
   ggctcccact ccatgaggta tttga 25
<210> 43
   <211> 35
   <212> DNA
   <213> Artificial Sequence
<220>
   <223> HLA-A group specific PCR primer A31 R
<400> 43
   cgcggagtgg tctcctggtc ccaatactca ggcat 35
<210> 44
   <211> 25
   <212> DNA
   <213> Artificial Sequence
<220>
   <223> HLA-A group specific PCR primer A3108 F
<400> 44
   ggctcccact ccatgaggta tttga 25
<210> 45
   <211> 19
   <212> DNA
   <213> Artificial Sequence
<220>
   <223> HLA-A group specific PCR primer A3108 R
<400> 45
   ttgtagtagc ggagcgcga 19
<210> 46
   <211> 19
   <212> DNA
   <213> Artificial Sequence
<220>
   <223> HLA-A group specific PCR primer A32 F
<400> 46
   cacgcagttc gtgcggtct 19
<210> 47
   <211> 19
   <212> DNA
   <213> Artificial Sequence
<220>
   <223> HLA-A group specific PCR primer A32 R
<400> 47
   ttgtagtagc ggagcgcga 19
<210> 48
   <211> 25
   <212> DNA
   <213> Artificial Sequence
<220>
   <223> HLA-A group specific PCR primer A3204 F
<400> 48
   gctcccactc catgaggtat ttgtt 25
<210> 49
   <211> 19
   <212> DNA
   <213> Artificial Sequence
<220>
   <223> HLA-A group specific PCR primer A3204 R
<400> 49
   ttgtagtagc ggagcgcga 19
<210> 50
   <211> 30
   <212> DNA
   <213> Artificial Sequence
<220>
   <223> HLA-A group specific PCR primer A34 F
<400> 50
   gcgggagcag gcgggtacca gcaggacgat 30
<210> 51
   <211> 30
   <212> DNA
   <213> Artificial Sequence
<220>
   <223> HLA-A group specific PCR primer A34 F2
<400> 51
   gcgggagcag tcgggtaccg gcaggacgat 30
<210> 52
   <211> 18
   <212> DNA
   <213> Artificial Sequence
<220>
   <223> HLA-A group specific PCR primer A34 R
<400> 52
   gagccactcc acgcacgt 18
<210> 53
   <211> 21
   <212> DNA
   <213> Artificial Sequence
<220>
   <223> HLA-A group specific PCR primer A4301 F
<400> 53
   ggccggagta ttgggaccta c 21
<210> 54
   <211> 23
   <212> DNA
   <213> Artificial Sequence
<220>
   <223> HLA-A group specific PCR primer A4301 R
<400> 54
   cgatgtaatc cttgccgtcg tca 23
<210> 55
   <211> 24
   <212> DNA
   <213> Artificial Sequence
<220>
   <223> HLA-A group specific PCR primer A66 F
<400> 55
   cccagtcaca gactgaccga gttg 24
<210> 56
   <211> 17
   <212> DNA
   <213> Artificial Sequence
<220>
   <223> HLA-A group specific PCR primer A66 R
<400> 56
   agccactcca cgcaccg 17
<210> 57
   <211> 19
   <212> DNA
   <213> Artificial Sequence
<220>
   <223> HLA-A group specific PCR primer A6602/03 F
<400> 57
   cgggtaccag caggacgat 19
<210> 58
   <211> 19
   <212> DNA
   <213> Artificial Sequence
<220>
   <223> HLA-A group specific PCR primer A6602/03 R
<400> 58
   ggagccactc cacgcactc 19
<210> 59
   <211> 19
   <212> DNA
   <213> Artificial Sequence
<220>
   <223> HLA-A group specific PCR primer A6901 F
<400> 59
   cggagtattg ggaccggaa 19
<210> 60
   <211> 16
   <212> DNA
   <213> Artificial Sequence
<220>
   <223> HLA-A group specific PCR primer A6901 R
<400> 60
   cgcggaggaa gcgcga 16
<210> 61
   <211> 19
   <212> DNA
   <213> Artificial Sequence
<220>
   <223> HLA-A group specific PCR primer A74 F
<400> 61
   cacgcagttc gtgcggtct 19
<210> 62
   <211> 19
   <212> DNA
   <213> Artificial Sequence
<220>
   <223> HLA-A group specific PCR primer A74 R
<400> 62
   cgcagggtcc ccaggtcta 19
<210> 63
   <211> 25
   <212> DNA
   <213> Artificial Sequence
<220>
   <223> HLA-A group specific PCR primer A8001 F
<400> 63
   gctcccactc catgaggtat ttgtt 25
<210> 64
   <211> 26
   <212> DNA
   <213> Artificial Sequence
<220>
   <223> HLA-A group specific PCR primer A8001 R
<400> 64
   gcgcccggcg gcgtcgctgt cgaaat 26